# EUROPEAN PATENT SPECIFICATION

(11) **EP 2 077 096 B1**
(45) Date of publication and mention of the grant of the patent: **12.10.2011**
(21) Application number: 09004463.7
(22) Date of filing: 10.08.2005
(51) Int. Cl.: A61B 19/08, A61B 19/02

(54) **Multiple ply sterilization wrap**
Mehrlagige Sterilisationshülle
Enveloppe de stérilisation de pli multiple

(30) Priority: 30.09.2004 US 955583
(43) Date of publication of application: 08.07.2009
(62) Divisional of application: 05785228.7
(73) Proprietor: KIMBERLY-CLARK WORLDWIDE, INC., Neenah, WI 54956 (US)
(72) Inventor: Mathis, Michael Peter, Marietta Georgia 30062 (US); Steindorf, Eric, Clayton, Roswell Georgia 30076 (US)
(74) Representative: Davies, Christopher Robert

(56) References cited:
- EP-A- 1 125 558
- EP-A- 1 192 958
- DE-A1- 4 031 943
- GB-A- 1 202 051
- GB-A- 1 399 884
- US-B1- 6 314 959

## Description

### BACKGROUND

Personnel in the Central Service Room (CSR) or the Sterile Processing Department (SPD) of hospitals are commonly charged with the responsibility of packaging surgical supplies to ensure that the sterility of the packaged contents are maintained all the way from sterilization to the point of reuse. Several activities are involved in the task of sterile supply delivery to the operating room and other units.

Much of the surgical instruments and supplies used in the operating room are reusable. These supplies typically include such things as clamps, scalpel blade handles, retractors, forceps, scissors, surgeons towels, basins and the like. All of these supplies must be collected after each procedure and sterilized before they can be used again in another procedure. To this end, the metal supplies are placed in stainless steel instrument trays, while soft goods such as surgeons towels, drapes, and gowns are wrapped directly. Per standard practices, the trays and package contents are each generally wrapped with two sheets of material commonly referred to as sterilization wrap.

The sterilization wrap is usually a woven or nonwoven material which when wrapped around the tray or package contents in a certain prescribed manner will permit the entry of sterilizing vapor/gas or other medium to sterilize the contents of the tray while denying the ingress of contaminants such as bacteria and other infectious causing materials or their vehicles after sterilization. Common means of sterilizing instruments include, among others, autoclaving with steam, exposure to ethylene oxide gas, and exposure to hydrogen peroxide plasma, as is done with the STERRAD® Sterilization System from Advanced Sterilization Products, Irvine, CA.

Using a wrapped tray as an example, once the wrapped tray and its contents have been sterilized, the wrapped tray is typically stored until it is needed for a surgical procedure. It is then transported to the point of use, typically an operating room. During storage and transfer to the operating room, the wrapped tray may be handled several different times. Each time the wrapped package is handled, there is a potential that the sterile nature of the package contents can be compromised. The two most common ways the wrapped package can be compromised are a tear or other breach of the wrapping material, and wetness or foreign ,materials identified on the outer wrapper. Either of which would warrant re-processing of the tray and contents.

In order to promote and maintain the sterility of the packaged contents, the Association of Operating Room Nurses (AORN) has developed certain recommended practices for the wrapping and handling of in-hospital processed packages. It is common practice among many hospitals as recommended by the AORN to "double wrap" in-hospital processed packages with two layers of barrier material. This minimizes the probability of a breach due to a flaw in any one layer of material.

A primary method of double wrapping is "sequential" in nature in that the package contents are first wrapped by one sheet of sterilization wrap and then wrapped again by another sheet of sterilization wrap. Another method of double wrapping is "simultaneous" in nature in that the package contents are wrapped by two sheets of sterilization wrap at the same time. That is, two sheets of sterilization wrap are aligned one on top of the other, and the item to be wrapped is placed on top of the two sheets, then the item is wrapped by both sheets of material at the same time.

Studies have been used to track packages from initial wrapping, all the way through sterilization, storage, handling, transfer, unwrapping and ultimate reuse. These studies indicate that the frequency of compromising wrapped items due to tears or holes has been declining because of improved handling and storage techniques and practices, as well as improved sterilization packaging products and materials. One of the main thrusts behind such efforts has been economics. Every time a sterile package is compromised, it must be taken out of circulation, unwrapped, rewrapped, and resterilized with a new sterilization wrapper before it can properly be reused. This wastes time and money.

While the frequency of compromising wrappers has been reduced thus resulting in the saving of time and money, the use of simultaneous wrapping techniques further increase the time savings in wrapping and opening packages and thus result in a still greater cost savings. Simultaneous wrapping takes less time than sequential wrapping and research in hospitals has shown simultaneous wrapping to be just as effective as sequential wrapping in maintaining sterility absent a breach in the wrap which is generally independent of the manner of wrapping.

Even though the hospital staff may desire to simultaneously wrap instead of sequentially wrap, the time it takes to set up the outer and inner sheet wrappers and the awkwardness of manipulating loose wrappers during simultaneous wrapping can offset the time savings hoped to be achieved when attempting to move away from sequential wrapping. Products have been developed that reduce the labor required in simultaneous wrapping by joining an outer and inner layer such that the layers can be manipulated as a unitary laminate wrapper. For example, one such product is KIMGUARD® ONE-STEP® produced by Kimberly-Clark Corporation which is described in-part in U.S. Patent No. 5,635,134 and 5,688,476. Other such two-ply sterilization wraps can be found U.S. Patent No. 6,406,764 to Bayer and U.S. Patent No. 6,517,916 to Bayer et al.

Whatever the material is that is being used as sterilization wrap, it is important that the wrapping materials provide good barrier properties to maintain package sterility and good strength properties so that tearing or other forms of breaching are held to a minimum. This has become a more important issue recently as th e average weight of trays being sterilized is increasing. Recent studies have shown that while tray weights in the past have generally been below 7.71 kg (17 pounds) tray weights over 11.34 kg (25 pounds) are now not uncommon, with some trays weighing over 35 pounds (15.88kg). This is a result of a shift to more instrument-intensive procedures, especially in areas of practice such as orthopedics and cardiovascular. For example, an orthopedic procedure tray may in clude multiples sizes of chisels, hammers and saws, as well as a blender with which to mix adhesive.

The heavier the tray and accompanying instruments become, the greater the possibility of tears and splits in the wrapping materials. The increase in forces that are exerted on the materials is directly proportional to the increase in weight of that which the material is wrapping. Materials made from polyolefins, such as polypropylene, are susceptible to pressure cuts due to polypropylene's characteristic of flowing under pressure. Increased pressure exerted by a tray, due to increased tray weight, creates a possibility of more pressure cuts. If the sterile package is compromised, it must be taken out of circulation, unwrapped, rewrapped, and resterilized before it can property be reused.

The strength of the two-ply systems has been increased with use of heavier layers or with stronger materials, but these solutions are accompanied by Increased costs. The inventors have found that there is an upper limit to strength that can be achieved through increasing the basis weight of a layer of polyolefin nonwoven material. Beyond this limit, the strength gains from Increased basis weight become marginal while the material becomes significantly more stiff, leading to difficulties in use of the sterilization wrap.

Such heavier and larger trays also provide other challenges to sterilization wrap systems. One problem often encountered with steam sterilization systems is that of wet packs. Wet packs often occur when not enough time is given to completely evaporate the condensate generated during the steam sterilization process. Wet packs are more common with heavy sterilization loads (i.e., more metal content) as more condensate is generated in the process of heating up the load. Condensate collects on and in the tray and if the cycle is not long enough to dry the wrapped package, such moisture will remain present in the tray and leave a potential path for microorganisms to breach the sterilization wrap. Standard procedure upon discovery of a wet pack is resterilization and thus increased time and cost to the end user. This problem is potentially even greater with the use of trays, as discussed above, that contain more instruments or physically larger trays, which means greater metal content.

Other challenges with larger trays also includes that fact that larger trays mean more surface area that is susceptible to exterior abrasions and cuts. Trays containing more items also create potential inventory issues and the need to notate all that the tray contains.

Consequently, there is a need for a new sterilization wrap system that actually reduces the likelihood of re-processing. Such a new sterilization wrap system would deliver sheets engineered for increased performance and increased functionality. Of particular interest is a sterilization wrap system that delivers higher strength than possible with current single-use sterilization wrap systems. Such attributes are provided by the present invention as will become more apparent upon a further review of the following specification, claims and drawings.

A prior art sterilizable surgical cloth is disclosed in DE-4031943.

### SUMMARY OF THE INVENTION

According to the present invention, there is provided a method as claimed in claim 1.

Each of the sheets of the inventive sterilization system may have a peripheral dimension. This dimension may be the same for each of the first, second and third sheets. Alternatively, the first and second sheets may have the same peripheral dimension, while the third sheet may have a different peripheral dimension. In one embodiment, the peripheral dimension of the third sheet is smaller than the peripheral dimension of the first and second sheets.

In one embodiment, the sheets of the sterilization wrap system are joined together using a bonding means such as adhesive bonding, stitch bonding, thermal bonding or ultrasonic bonding.

The sterilization wrap system may be adapted for use in steam sterilization or alternately may be adapted for use in ethylene oxide sterilization. In another embodiment the sterilization wrap system is adapted for forming a package by wrapping an article to be sterilized and then subsequently sterilizing the package.

In one embodiment of this method the sterilization conditions may be steam sterilization conditions or may be ethylene oxide sterilization conditions. In another embodiment of the method, the sterilization wrap system also includes a fourth sheet which is joined to the other three sheets.

### BRIEF DESCRIPTION OF THE DRAWINGS

FIG. 1 is a perspective view of a multiple ply, single step sterilization wrap system for use in the present invention with a sterilization tray ready for wrapping placed on top of the sterilization wrap system.
FIG. 2 is a cross-sectional side view of one embodiments of a multiple ply, single step sterilization wrap system for use in the present invention.
FIG. 3 is a cross-sectional side view of another embodiments of a multiple play, single step sterilization wrap system for use in the present invention.
FIG. 4 through 7 are top plan views of additional multiple ply, single step sterilization wrap systems for use in present invention with different bonding patterns for joining the separate sheets together.

### DETAILED DESCRIPTION

Disclosed herein is a sterilization wrap system suitable for use with simultaneous wrapping procedures for wrapping, sterilizing, storing and using sterilized items such as surgical supplies. While the present invention will be described in conjunction with its use in hospital and surgical room procedures, the sterilization wrap system of the present system is intended for use wherever there is a need for sterilized materials. Consequently, the following description of the present invention should not be considered a limitation as to the scope of use of the present invention.

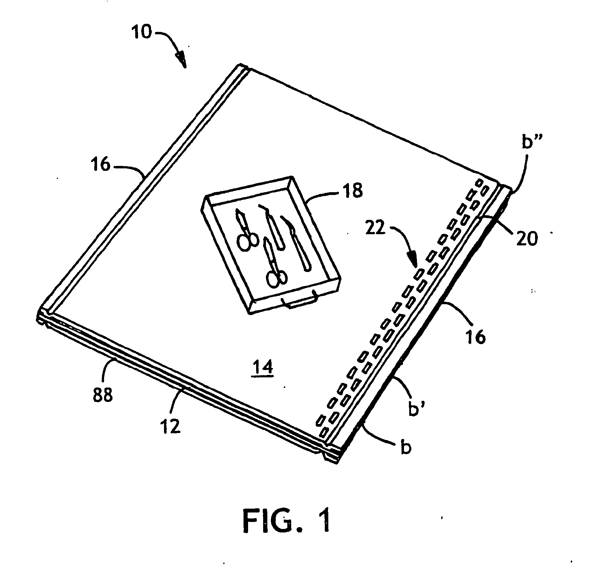

Referring to FIGS. 1, 2 and 3 of the drawings, there is shown a sterilization wrap system for containing and maintaining sterility of surgical supplies and the like. The sterilization wrap system 10 includes a first sheet 12, a second sheet 14, and a third functional sheet 88. As can be seen from FIG. 1, the first sheet 12, second sheet 14, and the third functional sheet 88 are placed in face to face relationship with one another, one on top of the other in vertical juxtaposition. The third sheet may be a single material or may also be a multilayer laminate, for example, such as a spunbond-meltblown-spunbond nonwoven laminate.

Generally, each of these sheets is of the same general size and shape. Most typically the sheets will be square or rectangular in shape. As a result, each sheet will have at least two generally parallel edges a,a',a" and b,b',b" located along their peripheries 16. It is also possible that the third functional sheet 88 may be a different size than that of the first and second sheets 12, 14. The third functional sheet may be larger or smaller than the first and second sheets. In the case where the third functional sheet 88 is smaller than the first and second sheet 12, 14, the third functional sheet 88 may be centered with respect to the first and second sheets 12,14 or may be positioned off-center as appropriate for Its functionality.

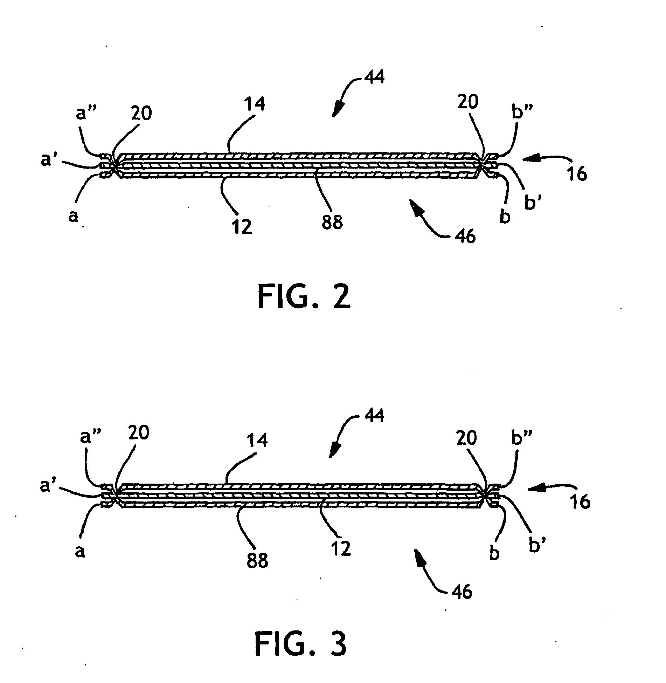

FIG. 2 shows a cross-sectional view of the invention shown in FIG. 1. In this embodiment, the outward facing surface of the second sheet 14 makes up the first exterior surface 44 of the sterilization wrap system 10. The outward facing surface of the first sheet 12 makes up the second exterior surface 46. The third functional sheet 88 is sandwiched between the first sheet 12 and the second sheet 14. In the embodiment shown in FIGS. 1 and 2, the wrapped item 18 would be in contact with the first exterior surface 44, which in this case would be the outward facing side of the second sheet 14. The second exterior surface 46, which in this case would be the outward facing side of the first sheet 12, would make up the outside of the finished wrapped sterilization package.

FIG. 3 shows a cross-sectional view of an alternative embodiment of the invention. In FIG. 3, the first exterior surface 44 would still be the outward facing surface of the second sheet 14, but the second exterior surface 46 would be the outward facing surface of the third functional sheet 88. In this embodiment the first sheet 12 would be sandwiched between the second sheet 14 and the third functional sheet 88.

Although not illustrated in the examples, all other combinations of these sheets are considered to be within the scope of this invention. As will be discussed in greater length below, additional functional sheets in various layering combinations with the sheets shown are also considered to be within the scope of this invention. Such additional functional sheets may be the same size as the first and second sheets 12, 14, may be the same size as the third functional sheet 88, or may be a different size than any of the other sheets.

To facilitate wrapping of an item 18 such as is shown in FIG. 1, the first sheet 12, and second sheet 14, and third functional sheet 88 are attached to one another in a manner so as to hold the three sheets together while still maintaining their visual distinctiveness so that the end user can visually see that the item is being wrapped by multiple separate sheets of sterilization wrap. Generally the sheets will be joined about all or a portion of their peripheries 16. As specifically shown in FIGS. 1, 2, and 3 the two sheets are joined to one another along the entire length of two generally parallel edges of the wrap, a-a'-a" and b-b'-b". The edges can be joined to one another by any number of suitable means including, but not limited to, adhesives, stitching, thermal bonding and ultrasonic bonding collectively referred to as joining. As shown in FIGS.1, 2, and 3 the continuous bonds 20 are perfected by ultrasonic bonding and run the entire length of the edges just interior to or along the periphery 16 on opposed sides of the sheets 12, 14 and 88.

In addition to or as an alternate to the continuous bonds 20, a second set of bonds 22 may be used to secure the multiple sheets together. The second set of bonds 22 in FIG. 1 are a series of spaced-apart and separate bond points in the form of two rows of parallel but spaced apart rectangles or other shapes with the rectangles in one row being offset from the other row so that they are in overlapping relationship if the sterilization wrap system 10 were viewed edge on. This bond pattern has been used to seam sleeves on disposable surgical gowns manufactured by the assignee of record, Kimberly-Clark Corporation of Neenah, Wisconsin. The second set of bonds 22 can be just interior of the continuous bonds 20 and serve to further join the multiple sheets 12, 14 and 88 together when used alone or in conjunction with the continuous bonds 20.

It is also conceivable that bonding is present on the entire periphery of the sheets, holding the sheets together at all four edges of the periphery.

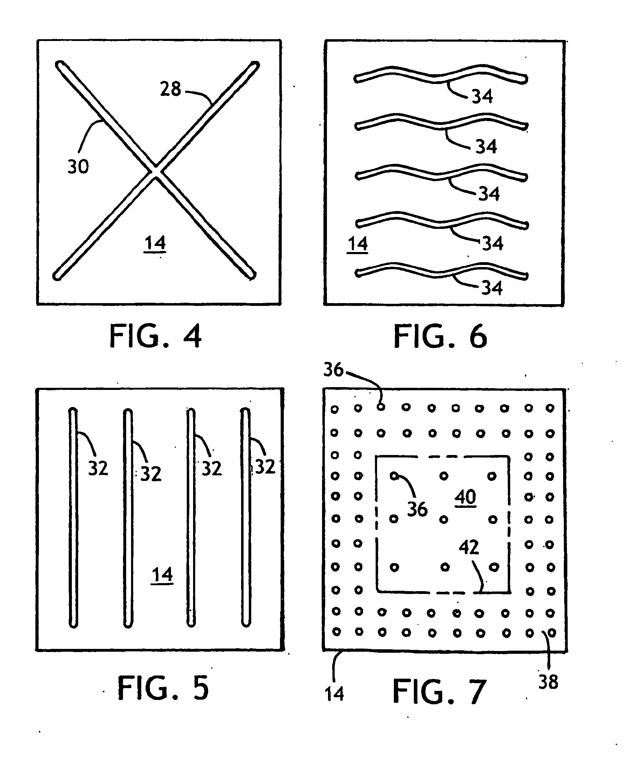

It also is possible to effect bonding between the multiple sheets 12, 14 and 88 in a variety of other manners which are exemplified, at least in part, in FIGS. 4 through 7. In FIGS. 4 through 7, the multiple sheets are superposed and joined to one another by one or more bond sites which may be long continuous bond lines such as are shown in FIGS. 4 through 6 or a plurality of localized bond points such as are shown in FIG. 7. In FIG. 4, which is a top plane view, the multiple wraps of the sterilization wrap system 10 are bonded together by two crisscrossing bond lines 28, 30 that form an "X"-pattern across the surface of the sterilization wrap system 10. In FIG. 5, the multiple sheets of the sterilization wrap system 10 are bonded to one another by a series of parallel bonds 32 that span all or a portion of the length or width of the sterilization wrap system 10. In FIG. 6, a series of sinusoidal bonds 34 are provided.

In addition to or in conjunction with the relatively long bonds or seams shown in FIGS. 4 through 6, the multiple sheets of the sterilization wrap system 10 may be joined by a plurality of localized, discontinuous bond points 36 such as are shown in FIG. 7. These bond points may be uniformly spaced across the surface of the sterilization wrap system 10 or they may be broken into two or more zones with each of these zones having varying degrees or densities of bond sites. Referring specifically to FIG. 7, the sterilization wrap system 10 is divided into a first zone 38 and a second zone 40 which, for purposes of illustration, are shown in FIG. 7 as being separated by an imaginary dashed line 42. The first zone 38 has a greater number of the overall plurality of bond sites per unit area than the second zone 40. In addition, the first zone 38 completely surrounds the second zone 40 thereby creating a sterilization wrap system 10 wherein the periphery of the sterilization wrap system 10 has a generally greater degree of bonding than the central portion of the sterilization wrap system 10.

Other combinations of bonding patterns can also be used. For example, indicia, logos and other printed matter can be used as the bond pattern to bond the multiple sheets of the wrap system together. Thus the bond pattern may be wording such as "KIMBERLY-CLARK" or "KIMGUARD®".

For some users, an important feature of the single step sterilization wrap system of the present invention is that the user can visually perceive that the system does in fact include multiple sheets. Being able to see this reinforces the comfort level of the user that not one but multiple sheets protect the wrapped item. Thus the multiple sheets of the sterilization wrap system should be joined to one another with a sufficient amount of bonding so that the sheets do not separate, but not with so much bonding that the sheets appear to be one. To this end, the sterilization wrap system 10 can be viewed as having a first exterior surface 44 and a second exterior surface 46 on opposed sides of the sterilization wrap system 10. See FIG. 2. To maintain the visual distinctiveness of the two respective surface areas it is advantageous if the surface area of the bond sites do not occupy more than about 50 percent of the surface area of either the first or second exterior surfaces 44, 46 of the sterilization wrap system 10.

While wishing to maintain the visual distinctiveness of the sterilization wrap system, the sheets of the sterilization wrap system should be sufficiently joined to one another so that they do not readily separate from one another throughout the process of removing the sterilization wrap system from its original packaging, wrapping the items to be sterilized with the sterilization wrap system and unwrapping the sterilized items for use. Consequently, it is desirable that there be at least a one pound tensile force needed to separate the joined sheets from one another.

Generally, the bonded sterilization wrap systems come in several sizes to wrap various size items and trays. Typical sizes include 18, 24, 30, 36, 40, 45, 48 and 54 inch square as well as 54 inch by 72 inch rectangular (where 1 inch = 2.54 cm). To wrap an item, in this case a sterilization tray 18 such as shown in FIG. 1, the item is placed on top of the sterilization wrap system 10 in contact with the second sheet 14 such that the four corners of the sterilization wrap system can be folded over onto the package one at a time. Once the folding is completed, the sterilization wrap system is sealed with tape and the wrapped package is ready to be sterilized.

Each of the sheets can have its own special characteristics. The main function of the second sheet 14 is to act as the primary filtration barrier while the primary function of the first sheet 12 is to provide strength with a secondary function of also providing a barrier to bacteria and other contaminants. The third functional sheet 88 provides an additional functionality either not present in the first or second sheets 12, 14 or provides a functionality to a greater degree than present in the first or second sheets 12, 14.

Both the first sheet 12 and the second sheet 14 can be made from a number of materials. Sterilization wrap systems are generally characterized as falling into two main classes, reusables and disposables. Reusables are materials which, as the name suggests, can be reused, typically by washing or some other form of cleaning. Disposables, on the other hand, are usually one-use items which are discarded or recycled after their initial use. Generally, cloth, linen or other woven materials fall into the reusable category while disposables normally include nonwoven materials made from either or both natural and synthetic fibers such as paper, fibrous polymeric nonwovens as well as films which are capable of passing sterilants and retarding transmission of bacteria and other contaminants.

Nonwoven sterilization wrap systems have become particularly well-liked due to their barrier properties, economics and consistent quality. The nonwoven materials can be made from a variety of processes including, but not limited to, air laying processes, wet laid processes, hydroentangling processes, spunbonding, meltblowing, staple fiber carding and bonding, and solution spinning. The fibers themselves can be made from a variety of both natural and synthetic materials including, but not limited to, cellulose, rayon, nylon, polyesters, polyolefins and many other materials The fibers may be relatively short, staple length fibers, typically less than 7.62 cm (3 inches) or longer more continuous fibers such as are produced by spunbonding and meltblowing processes. Whatever materials are chosen, the resultant wrap must be compatible with the particular sterilization technique being used and must also provide both strength and barrier properties to maintain the sterile nature of the wrapped contents until use.

It has been found that polyolefin-based fibers and their resultant nonwovens are particularly well-suited for the production of sterilization wrap systems. Polypropylene spunbonded nonwovens such as are produced by the Assignee of record, Kimberly-Clark Corporation, can be used to impart strength characteristics to the sterilization wrap and in particular, the first sheet 12. The first sheet 12 is made from a laminate of spunbonded, meltblown, spunbonded to impart both strength and barrier properties to the first sheet 12.

A spunbonded-meltblown-spunbonded material is made from three separate layers which are laminated to one another. The method of making these layers is known and described in commonly assigned U.S. Pat. No. 4,041,203 to Brock et al. The material of Brock et al is a three layer laminate of spunbonded-meltblown-spunbonded layers which is also commonly referred to by the acronym "SMS". The two outer layers of SMS are a spunbonded material made from extruded polyolefin fibers, or filaments, laid down in a random pattern and then bonded to one another. The inner layer is a meltblown layer also made from extruded polyolefin fibers generally of a smaller diameter than the fibers in the spunbonded layers. As a result, the meltblown layer provides increased barrier properties due to it fine fiber structure which permits the sterilizing agent to pass through the fabric while preventing passage of bacteria and other contaminants. Conversely, the two outer spunbonded layers provide a greater portion of the strength factor in the overall laminate. The laminate may be prepared using an intermittent bond pattern that is preferably employed with the pattern being substantially regularly repeating over the surface of the laminate. The pattern is selected such that the bonds occupy about 5-50% of the surface area of the laminate. Desirably, the bonds occupy about 10-30% of the surface area of the laminate.

A particular feature of the present invention is the specific tailoring available for each of the layers in the respective first sheet 12 and second sheet 14. While the two sheets can be identical to one another, in more refined embodiments of the present invention the first sheet 12 is designed to have higher strength properties than the second sheet 14. This is to provide a stronger barrier to tears and other possible breaches of the wrapped item from exterior objects. Conversely, in more refined embodiments of the present invention, the second sheet 14 is designed to have higher barrier properties than the first sheet 12. Adjusting the barrier and strength properties can generally be accomplished by adjusting the basis weights of the outer and inner sheets as well as the basis weights of each of the individual layers within each of the sheets. Suitable basis weight ranges for either of the sheets range between 17 to about 119 grams per square meter (gsm)) (about 0.5 and about 3.5 ounces per square yard (osy)).

While the first and second wrap sheets 12, 14 provide barrier functionality and some degree of strength to the sterilization wrap system, the third functional sheet 88, provides additional functionality to the wrap system. One possible functionality is a higher degree of strength than is provided by the first and second sheets 12, 14. One of the limitations of using a nonwoven material such as a polypropylene spunbond-meltblown-spunbond of the type often used for the first and second sheets is that there a limitation of strength that can be provided by increasing the basis weight of the laminate. The strength of the laminate plateaus as the laminate becomes thicker and the laminate becomes more difficult to bond together. The resulting laminate is stiff and becomes inappropriate for use as a sheet of the sterilization wrap system. However, the third functional sheet 88, may be made of a material of a higher strength without any concern for barrier properties already provided by the first and second sheets. Such a third functional sheet 88 may be a woven or nonwoven fabric of a stronger polymer, for example, such as polyester or nylon. When such a third functional sheet with increased strength is incorporated into the sterilization wrap system, this third functional sheet 88 acts as the strength-bearing member of the sterilization wrap system. Without having to provide all of the strength, the basis weight of the first and second sheets 12, 14 may be reduced, providing for some savings to offset the cost of additional sheets.

Additionally, inventory and manufacturing could be simplified by using common first and second sheets 12, 14 across an entire, or minimally across a portion of, a sterilization wrap system product line. A full sterilization wrap system product line will usually have different strength sterilization wrap systems available for consumers various needs. This has meant that various weights of first and second sheets had to be produced and kept in inventory to meet the requirements of the various products offered to the consumers. However, with the multiple sheet sterilization wrap system of the present invention, the same weight first and second sheets 12, 14 could be used across the product line. Additional, strength, barrier, or other functionalities that are associated with the various products of the product line would be added by including one or more additional sheets.

For example, product line of increasing strength which included a wrap system made of two sheets of 17 gsm (0.5 osy)) SMS, a wrap system made of two sheet of 35 gsm (1.0 osy) SMS, and a wrap made of two sheets of 52 gsm (1.5 osy) SMS would have required the production and inventory of 0.5, 1.0 and 1.5 osy SMS materials. The same product line utilizing the present invention could be produced with multiple sheets of 0.5 osy SMS material or with multiple sheets of 0.5 osy SMS along with an additional sheet(s) of increased strength functionality. With the present invention, fewer different grades of sheet materials may be manufactured and kept in inventory to produce the current sterilization wrap system product line, in addition to sterilization wrap systems of increased functionality as discussed elsewhere in this description.

Another functionality that may be provided is abrasion and or cut resistance. As discussed earlier, the advent of larger sterilization trays, can mean a larger surface area susceptible to abrading, pilling, scratches and cuts. Such defects detected on the outside of the sterilization pack can mean that the tray needs to be re-sterilized. The third functional sheet 88 may be the outer wrap of the sterilization wrap system and may be designed to provide a strong material, resistant to such abrasion or cuts. If a material such as a polyester or nylon is used as the outer sheet, this third functional sheet 88 may provide the increased strength benefit discussed above, along with an abrasion- or cut-resistant functionality.

The third functional sheet 88 may also be designed to address the problem of wetpacks. The sheet may incorporate an absorbent material such as a cellulose, pulp, cotton, other naturally absorbent fibers, or fibers that have been treated to be hydrophilic. If placed as the inside layer of the sterilization wrap system, the sheet may help absorb and spread out condensation produced in the sterilization process, thus enabling the moisture to evaporate more efficiently in the drying phase of the sterilization process.

Other functionalities may be incorporated into the third functional sheet 88 in combination with the functionalities already discussed or may be present on their own. Some of the functionalities considered include improved slip-resistance, a surface that is easy to print on, a surface that is easy to write on, and/or a sterility indicator. Additional functionalities considered are materials that are more receptive to fastening tape, hook and loop materials, self-closing materials, and/or other means of fastening the sterilization wrap system closed. Any of these functionalities in combination, or on their own, may be designed into a single material to be used as the third functional sheet 88.

Alternatively, additional functional sheets may be incorporated into the sterilization wrap system to provide their unique functionality or multiple functionalities. For example, the sterilization wrap system may include a fourth functional sheet, where the fourth functional sheet has a different functionality than the other three sheets. Alternatively, the sterilization wrap system may also include a fifth functional sheet. Additional functional sheets are also contemplated as within the scope of this invention.

Some of such additional functional sheets may be the same size of the first and second wrap sheets or they may be of a different size that better suits their particular functionality. For example, an additional functional sheet incorporating the functionality of hook and loop closures may only be present on a portion of a functional sheet where appropriate to make a closure when the sterilization wrap system is wrapped around an item. Another example would be a functional sheet with an absorbance functionality that would make up the inside (in contact with the item to be sterilized) of the sterilization wrap system. Such an absorbent sheet may be smaller than the dimension of the first and second sheets 12, 14 and centered with respect to the first and second sheets 12, 14. This would put absorbent fibers in contact with the item to be sterilized to reduce wet packs, but would not use absorbent fibers in areas where they would not be useful.

When designing inner and outer sheets with different properties it is usually important that sterilization wrap system 10 be positioned such that proper sheet surface faces the item to be wrapped and the other sheet surface faces away from the wrapped item. Typically this will mean that the first exterior surface 44 is in contact with the item 18 to be wrapped and the second exterior surface will be positioned away from the wrapped item 18. To this end it may be desirable to produce Inner and outer sheets which are visually distinguishable from one another. By "visually distinguishable" it is meant that a majority of people who routinely use such materials would be able to tell the difference between the first exterior surface 44 and the second exterior surface 46 of the sterilization wrap system 10 based upon a visual observation of the two surfaces. One means of achieving this would be shading, coloring or adding a texture to the second sheet 14 differently than the first sheet 12. In addition, printing or other indicia may be used to differentiate the two sheets from one another.

Sheets having different functionalities could also be made to be visually distinct. By "visually distinct" it is meant that sheets having a particular functionality would be visually distinguishable from sheets having a different associated functionality. As such, certain functionalities could be associated with a particular visually distinction. For example, a sheet having slip resistance functionality may be a particular color while a sheet with high strength functionality may be a different color, or may be differently textured, than the slip resistant sheet.

An array of sterilization wrap systems may be made with different combinations of functionalities as embodied in their respective sheets. As discussed above, each of the different functionalities as embodied as their respective sheets may be visually distinct from other sheets of differing functionality. Information regarding these functionalities along with their associated visual distinctions may accompany the array of sterilization wrap systems. A person using this information would then be able to select from among the array of available sterilization wrap systems to select the functionalities that they desire.

In one embodiment, the sterilization wrap system may be made up of four sheets, each with a different functionality, bound together solely at one of the corners of the sheets. A person using information regarding the functionality of the various sheets would be able to select the desired functionality and placement of that functionality relative to the item to be sterilized. The person would be able to manipulate the sheets of the sterilization wrap system such that the sheets with the selected functionalities would be oriented as desired. This manipulation of sheets may include, but is not limited to, the ability to fold, tear off, remove portions, twist, roll-up, shift, or flip over any one or more sheets of a wrap system and any combination of manipulations thereof.

A further embodiment would have a fifth functional sheet would also be attached similarly at the corner of the sterilization wrap system. Other embodiments with a sixth, seventh, and/or greater numbers of functional sheets may also be used.

A further embodiment of the above sterilization wrap system with multiple functional sheets would use other means of attachment of the sheets such that the desired functionality could be chosen from among the functional sheets. Rather than bonding the sheets together solely at the corner of the sheets, the sheets could be bonded at opposite corners, or at three corners, or any other bonding pattern that would allow manipulation of the sheets such as to allow rearrangement of the sheets relative to the item to be sterilized. One further embodiment may be to bond the sheets together in such a manner that sheets of unwanted functionality could easily be removed from the sterilization wrap system, leaving only those sheets with functionalities that are desired.

### EXAMPLES

To demonstrate the attributes possible with the present invention, several sterilization wrap systems were prepared and then tested against other currently available sterilization wraps. Kimberly-Clark Corporation, the assignee of record, manufactures a series of single sheet and dual sheet sterilization wrap materials made from a series of SMS laminates of various basis weights. Some of these base SMS laminates, along with their respective basis weights (given in units of grams per square meter) are shown in Table 1.

**TABLE 1**

| **Material** | **Basis Weight (gsm)** |
|---|---|
| KC100 | 35.6 |
| KC300 | 42.4 |
| KC400 | 59.3 |
| KC500 | 69.5 |

Several multiple-sheet sterilization wrap systems were produced for testing using these standard base SMS materials. For comparison, sterilization wrap systems commercially produced were duplicated by combining two sheets of each of the materials listed in Table 1 (Codes 3 - 5). Codes demonstrating the increased strength functionality embodiment of the present invention were produced by adding an additional strength sheet between two sheets of the standard base SMS material, KC300. A list and description of each of the codes produced for testing is given in Table 2.

**TABLE 2**

| **Code** | **Materials (in sheet order)** | **Total basis weight (gsm)** |
|---|---|---|
| Code 1 | KC300/ PP SB / KC300 | 115.3 |
| Code 2 | KC300 / hydroentangled SB / KC 300 | 128.9 |
| Code 3 | KC300 / KC300 | 84.8 |
| Code 4 | KC400 / KC400 | 118.7 |
| Code 5 | KC500 / KC500 | 139.0 |

Code 1 comprised a sheet of polypropylene (PP) spunbond (SB) material, having a basis weight of 30.52 gsm, sandwiched between two sheets of KC300 base SMS material. Code 2 comprised a sheet of PP hydroentangled SB, having a basis weight of 44.08 gsm, sandwiched between two sheets of KC300 base SMS material. Codes 3, 4 and 5 are sterilization wrap systems each made of two sheets of the SMS base materials as shown in Table 2. Codes 3, 4 and 5 are commercially available as KIMGUARD ONE-STEP® KC300, KIMGUARD ONE-STEP® KC400, and KIMGUARD ONE-STEP® KC500, respectively.

Each of the samples was tested for grab tensile strength, trapezoidal tear strength, air permeability, and drape stiffness. For all of the tests, except the air permeability test, the material was tested with samples taken from both the machine-direction (MD) and the cross-direction (CD) of the material. The term "machine-direction" as used here refers to the direction of travel of the forming surface onto which fibers are deposited during formation of the nonwoven web. The term 'cross-direction" as used here refers to the direction that is perpendicular to the machine-direction defined above.

Grab tensile testing measures the strength of a material, in a single direction of the material, by measuring the load required to break the material under constant elongation. Grab tensile strengths of the samples were measured essentially in accordance with ASTM 5034-95 (using dry samples). Tensile strength measurements of samples were made with a Constant-Rate-Of-Extension (CRE) Testing Machine, namely a Sintech S/2 Workstation, from MTS Systems Corporation, Eden Prairie, MN, USA, equipped with either a 22.68kg (50 lbs) or a 45-36kg (100 lbs) load cell (i.e., the larger load cell used for stronger samples) and using Testworks 4 software, also from MTS Systems Corporation. "Tensile strength" refers to the maximum load or force (i.e., breaking force) encountered while elongating the sample to break. The results are expressed in units of force (lbs-force; also referred to in this document as "lbs.") and are the average of 10 individual samples, each measuring 102 mm (4 inches) wide by 152 mm (6 inches) long (extension direction). To convert the results given in pounds to kilograms, multiply by 0.454.

Trapezoidal tear strength is a measure of the resistance to tear propagation of materials. Trapezoidal tear strength was measured essentially in accordance with ASTM D5733-99 (Condition 1 - no conditioning). Trapezoidal tear strength measurements of samples were made with a Constant-Rate-Of-Extension (CRE) Testing Machine, namely a Sintech S/2 Workstation, from MTS Systems Corporation, Eden Prairie; MN, USA, equipped with either a 11.35kg (25 lbs) load cell and using Testworks 4 software, also from MTS Systems Corporation. Ten individual samples of each code were tested where each sample was die cut to produce the isosceles trapezoid sample required by the test method. The tear strength was calculated as the average of the first peak and the peak load and is expressed in units of force (lbs-force). To convert the results given in pounds to kilograms, multiply by 0.454.

Air permeability (porosity) is a measure of the rate of air-flow through a known specimen area. The higher the result reading, the more open the material is, thus allowing more air to pass through. Air permeability was measured essentially in accordance with ASTM 737-96. Measurements were made of the sample using a TEXTEST FX 3300 Air Permeability Tester, available from Schmid Corporation of Spartanburg, SC, USA. The test head used was 38 cm² and the test pressure provided was 125 Pa. The testing lab conditions were 23 ± 1° C and 50 ± 2% RH. The permeability was calculated as the average of 10 individual samples and is expressed in cubic feet per minute (cfm). To convert the results given in cubic feet per minute to cubic meters per minute, multiply by 0.0283.

Drape stiffness is a measure of the resistance to bending for a material. Drape stiffness was measured essentially in accordance with the Cantilever Test (Option A) of ASTM 1388-96 (2002). The material stiffness was tested using a Cantilever Test Apparatus, namely a Model 79-10 Cantilever Bending Tester, available from Testing Machines, Inc. of Amityville, NY, USA. The value reported as "drape stiffness" is one-half of the length of material overhanging the edge of the testing platform when the leading edge of the material reaches an angle of 41.5° from horizontal. The drape stiffness was calculated as the average of 10 individual samples and is expressed in length (in.). To convert the results given in inches to millimeters, multiply by 25.4.

The sterilization wrap systems used in testing were made of multiple sheets, where the sheets were bound together on two opposing edges of the sterilization wrap system. For drape stiffness testing, each individual sample measured 25 mm wide by 203 mm long (1 inch by 8 inches). As with grab tensile and trapezoidal tear testing, sets of samples were tested in the MD and CD of the sterilization wrap systems, not including the area where the sheets were bound together. These samples are reported as "unbound" in **Table 3.** An additional sample set was taken in the CD of the sterilization wrap systems in such a way to include the bound edge of the wrap systems. This bound edge for these samples was used as the leading edge of the sample in the drape stiffness test and the results of such sample sets are reported as "bound" in Table 3.

The results of the testing are given in Table 3.

**TABLE 3**

| **Test** | **Code 1** | **Code 2** | **Code 3** | **Code 4** | **Code 5** |
|---|---|---|---|---|---|
| Grab Tensile-CD (lbs.-force) | 41.55 | 50.9 | 35 | 55.21 | 65.9 |
| Grab Tensile-MD (lbs.-force) | 41.09 | 68.7 | 32.8 | 54.46 | 64.3 |
| Trapezoidal Tear - CD (lbs.-force) | 12.1 | 18 | 9.9 | 18 | 18.1 |
| Trapezoidal Tear- MD (lbs.-force) | 11.2 | 23.1 | 8.4 | 17.3 | 16.7 |
| Air Permeability (cfm) | 34.79 | 31.04 | 33.8 | 29.19 | 23 |
| Drape Stiffness - CD (in.) (unbound) | 8.865 | 6.92 | 5.285 | 6.985 | 7.425 |
| Drape Stiffness - MD (in.) (unbound) | 7.915 | 8.305 | 6.79 | 8.57 | 8.865 |
| Drape Stiffness - CD (in.) (bound) | 7.105 | 6.81 | 5.545 | 6.815 | 7.345 |

As can be seen from Table 3, the strength (grab tensile) and tear resistance (trapezoidal tear) of Code 1 falls between the comparative values for codes 3 and 4. This is expected because Code 1 is the same as Code 3 except for the addition of a sheet of polypropylene (PP) spunbond material in Code 1. The base layers of Codes 1, 2 and 3 are sheets of PP SMS material, which again is a three layer laminate of PP spunbonded-meltblown-spunbonded layers. It would be expected that the addition of a PP spunbond sheet to sheets of SMS materials would function similarly to a mere increase in the basis weight of the SMS sheets alone. Comparing Codes 3, 4 and 5 in Table 3, it can be seen that as the basis weight of the SMS increases, the strength of the wrap system increases. The strength of Code 1 falls between Codes 3 and 4, as a mere increase in SMS basis weight would suggest.

The addition of the strength functionality of the invention is partially shown by Code 2 in Table 3. Code 2 was the same as Code 3 except for the addition of a sheet of hydroentangled PP spunbond material in Code 2. The total basis weight of Code 2 was between that of Code 4 and Code 5. An increase in strength due to an increase in basis weight would suggest strength of Code 2 to be between that of Codes 4 and 5. However, the strength and tear resistance of Code 2 in the MD was noticeably higher than that of both Codes 4 and 5.Testing of Code 2 in the CD was similar, or slightly lower, than that of Code 4. Code 2 also had greater air permeability and lower drape stiffness than both Code 4 and Code 5.

Two additional codes demonstrating the increased strength functionality embodiment of the present invention were produced by adding a nylon strength sheet between two sheets of the standard base SMS material, KC100. A list and description of each of these codes is given in Table 4.

**TABLE4**

| **Code** | **Materials (in sheet order)** | **Total basis weight (gsm)** |
|---|---|---|
| Code 6 | KC100/ NYLON 1 / KC100 | 139.0 |
| Code 7 | KC100 / NYLON 2/ KC 100 | 139.0 |

Code 6 comprised a sheet of the Nylon 1 material sandwiched between two sheets of KC100 base SMS material. The Nylon 1 material was a 67.8 gsm sheet of thermally bonded spunbond nylon material with trilobal nylon filaments available under the ORION® trade designation from CEREX Advanced Fabrics, Inc., Cantonment, FL, USA. Code 7. comprised a sheet of Nylon 2 material sandwiched between two sheets of KC100 base SMS material. The Nylon 2 material was a 67.8 gsm sheet of autogenously bonded continuous filament nylon material available under the CEREX® trade designation from CEREX Advanced Fabrics, Inc., Cantonment, FL, USA. The base SMS material used in codes 6 and 7 was the lighter KC100 material (rather than the KC300 used in Codes 1-2) to provide a wrap system having a total basis weight comparable to the wrap system of Code 5. Code 5 was made of two sheets of the KC500 SMS base materials and, as can been seen in Table 3, was the strongest sterilization wrap system among Codes 1 to 5.

Codes 6 and 7 were tested for grab tensile strength In the MD in the same manner that the testing of Codes 1-5 was conducted. The results of the testing are given in Table 5.

**TABLE 5**

| **Test** | **Code 6** | **Code 7** |
|---|---|---|
| Grab Tensile-MD (lbs.-force) | 67.7 | 80.4 |

As can be seen in comparing the results of Codes 6 and 7 in Table 5 with the similar test results of Code 5 in Table 3, the addition of the nylon materials as a functional strength layer increased the strength of the sterilization wrap system- While all three codes had similar overall basis weights, the grab tensile was higher for the codes containing the nylon functional strength layer even though the basis weight of the base SMS materials for the same codes was much lower. The level of strength of the sterilization wrap systems of Codes 6 and 7 was very similar to the level of strength of the individual nylon materials as reported by the manufacturer.

Therefore, additional functionality can be added to a sterilization wrap system with the inclusion of one or more functional sheets. As the examples have demonstrated, one such possibility is the addition of strength functionality sheet to increase the strength of the sterilization wrap system to a greater degree than is possible by merely increasing the basis weight of the base SMS sheets. A stronger sterilization wrap system can be produced which is easy to manipulate and provides the necessary barrier properties. Likewise, using the description of this invention, one skilled in the art can produce sterilization wrap systems with addition functionalities or combinations of functionalities.

## Claims

1. A method of sterilizing an article (18) comprising:
providing an article (18); providing a sterilization wrap system (10), where the sterilization wrap system (10) comprises (a) a first sheet (12) comprising a laminate having a spunbonded layer, a meltblown layer, and a spunbonded layer, (b) a second sheet (14) comprising a laminate having a spunbonded layer, a meltblown layer, and a spunbonded layer and (c) a third said sterilization wrap system (10); and sheet (88); where the first, second and third sheets are placed in face to face relationship with one another and joined together ;
wrapping the article (18) with said sterilization wrap system (10); and
exposing the wrapped article (18) to sterilizing conditions for a sufficient time to substantially sterilize the article (18).

2. The method of claim 1, where the sterilizing conditions are selected from steam sterilizing conditions and ethylene oxide sterilizing conditions.

## Patentansprüche

1. Verfahren zum Sterilisieren eines Artikels (18), umfassend:
Zurverfügungstellen eines Artikels;
Zurverfügungstellen eines Sterilisierungseinwickelsystems (10), wobei das Sterilisierungseinwickelsystem (10) (a) eine erste Bahn (12) enthaltend ein Laminat mit einer Spunbonded-Schicht, einer schmelzgeblasenen Schicht und einer Spunbonded-Schicht, (b) eine zweite Bahn (14) enthaltend ein Laminat mit einer Spunbonded-Schicht, einer schmelzgeblasenen Schicht und einer Spunbonded-Schicht und (c) eine dritte Bahn (88) aufweist; wobei die erste, zweite und dritte Bahn werden in vertrauliches Verhältnis miteinander gelegt und miteinander verbunden sind;
Einwickeln des Artikels (18) mit dem Sterilisierungseinwickelsystem (10); und Aussetzen des eingewickelten Artikels (18) von Sterilisierungsbedingungen für eine Zeit, die ausreicht, um den Artikel (18) im Wesentlichen zu sterilisieren.

2. Verfahren nach Anspruch 1, wobei die Sterilisierungsbedingungen ausgewählt sind aus Dampfsterilisierungsbedingungen und Ethylenoxidsterilisierungsbedingungen.

## Revendications

1. Procédé de stérilisation d'un article (18), comprenant :
la fourniture d'un article (18) ;
la fourniture d'un système d'enveloppement pour stérilisation (10), dans lequel le système d'enveloppement pour stérilisation (10) comprend (a) une première feuille (12) comprenant un stratifié ayant une couche obtenue par filage-nappage, une couche obtenue par extrusion-soufflage et une couche obtenue par filage-nappage, (b) une seconde feuille (14) comprenant un stratifié ayant une couche obtenue par filage-nappage, une couche obtenue par extrusion-soufflage et une couche obtenue par filage-nappage, et (c) une troisième feuille (88), les première, seconde et troisième feuilles étant placées en relation face à face l'une par rapport à l'autre et réunies ensemble ;
l'enveloppement de l'article (18) avec ledit système d'enveloppement pour stérilisation (10) ; et
l'exposition de l'article enveloppé (18) à des conditions de stérilisation pendant une durée suffisante pour stériliser substantiellement l'article (18).

2. Procédé selon la revendication 1, dans lequel les conditions de stérilisation sont sélectionnées entre des conditions de stérilisation à la vapeur et des conditions de stérilisation à l'oxyde d'éthylène.
